# FASCICULE DE BREVET EUROPEEN

(11) **EP 0 521 095 B1**
(45) Date de publication et mention de la délivrance du brevet: **09.11.1994**
(21) Numéro de dépôt: 91907237.1
(22) Date de dépôt: 19.03.1991
(51) Int. Cl.: A61H 15/00, A61H 9/00

(54) **APPAREIL DE MASSAGE**
MASSAGEGERÄT
MASSAGE DEVICE

(30) Priorité: 20.03.1990 FR 9003981
(43) Date de publication de la demande: 07.01.1993
(73) Titulaire: KARAGOZIAN, Serge, F-26000 Valence (FR); FUMAS, Claude, F-26500 Bourg-Les-Valence (FR); COLOMBAT, Jean-Jacques, F-26540 Mours-Saint-Eusèbe (FR)
(72) Inventeur: KARAGOZIAN, Serge, F-26000 Valence (FR); FUMAS, Claude, F-26500 Bourg-Les-Valence (FR); COLOMBAT, Jean-Jacques, F-26540 Mours-Saint-Eusèbe (FR)
(74) Mandataire: Guerre, Dominique
(86) Numéro de dépôt international: FR9100222
(87) Numéro de publication internationale: WO9114417

(56) Documents cités:
- EP-A- 284 527
- FR-A- 854 937
- FR-A- 2 483 225

## Description

La présente invention concerne un appareil destiné au massage du tissu conjonctif cutané et sous-cutané, à des fins de diagnostic, thérapeutiques, antalgiques, notamment dermalgie réflexe, ou dermo-esthétiques. Cet appareil peut être aussi utilisé selon différentes techniques opératoires ou de manipulation, bien connues en médecine physique ou kinésithérapie.

Conformément au document FR-C-854 937, on a décrit un appareil de massage travaillant par décollement de la peau par pression. Cet appareil comprend une tête de traitement manipulée par l'opérateur à la surface du corps, comportant une chambre d'aspiration en forme d'alvéole ou cylindrique, reliée a un moyen d'aspiration ou source de dépression, pour aspirer dans cette chambre un pli cutané en forme de cloche. Cette chambre d'aspiration communique vers la peau par une ouverture délimitée par une paroi tronconique. La bordure de cette ouverture est pourvue d'une multitude de billes, régulièrement distribuées côte à côte dans un même plan équatorial, sur le pourtour de l'ouverture, chaque bille étant fixe en position par rapport au boîtier, mais montée libre en rotation dans toutes les directions sur cette bordure. Le diamètre de ces billes demeure du même ordre de grandeur que l'épaisseur de la paroi tronconique, de telle sorte que le niveau inférieur des billes, déterminant la surface de roulement de la tête de traitement est situé à distance tout en étant très proche du bord libre de l'ouverture. En fait, les billes montrées par le document FR-C-854 937 ne peuvent servir au massage de la peau, et n'ont d'autre fonction que d'assurer un déplacement sans frottement de la tête de traitement à la surface de la peau.

Selon le document FR-C-854 937, le seul massage obtenu résulte du déplacement, par exemple en rond, de la tête de traitement à la surface de la peau, et du déplacement corrélatif du pli cutané en forme de cloche sur le corps, avec la pliure correspondante en mouvement de la peau. Mais, compte tenu de la faible épaisseur de la bordure d'aspiration, que ne compensent pas les billes de roulement, cette pliure intervient nécessairement avec une cassure importante de la peau, donc traumatisante, et génératrice de plicatures en forme de sillons.

Conformément au document FR-C-1 225 094, on a décrit une tête de massage à boules, manipulée par l'opérateur à la surface du corps, comportant un corps incorporant une réserve centrale pour un produit de traitement, et une pluralité de boules, distribuées sur le pourtour dudit corps, fixes en position par rapport audit corps, mais montées libres en rotation dans toutes les directions sur ledit corps. Ces boules sont séparées par des intervalles sensiblement égaux. Le niveau inférieur des boules, déterminant la surface de roulement de la tête de massage, est situé au-dessous du bord libre du corps, à une distance supérieure au rayon des boules.

Selon le document FR-C-1 225 094, le seul massage obtenu résulte de la pression de la tête de massage sur la surface de la peau, avec la compression correspondante de l'épiderme, du derme, et de l'hypoderme. En pratique, une telle compression s'avère défavorable à tout effet bénéfique, car elle peut être traumatisante au niveau vasculaire, en écrasant la lumière des capillaires de la peau.

La présente invention a pour objet un appareil de massage par dépression, travaillant donc par décollement de la peau, du type décrit par le document FR-C-854 937. Plus précisément, l'invention a pour objet un appareil permettant de décoller toutes les couches de la peau, pour les masser effectivement dans leur conformation décollée en cloche, et ce avec une grande facilité pour l'opérateur, et sans traumatisme pour le patient.

Selon la présente invention, des interstices de pénétration partielle des faces internes du pli cutané en cloche sont ménagés et distribués sur le pourtour de l'ouverture 2a de la chambre 2 d'aspiration, et sont définis, entre la surface de roulement et le bord libre de l'ouverture, par des intervalles sensiblement égaux séparant les billes 5 dans leur plan équatorial.

Grâce à la disposition des billes par rapport au bord libre de l'ouverture d'aspiration, selon l'invention, il devient possible de masser la peau, non seulement à la base de la déformation cutanée en cloche, mais aussi sur les faces internes du pli en cloche. En tournant sur elles-mêmes, par rapport au pli en cloche, les billes crèent une stimulation douce, mécanique, vasculaire et réflexe, à la condition de ne pas appliquer une pression axiale trop importante sur la tête de traitement.

Par ailleurs, le mouvement circulaire large appliqué à la tête de traitement, en liaison avec l'action des billes, permet un effet de chasse vasculaire intense, tout en respectant les constituants du tissu conjonctif cutané et vasculaire.

Comme montré ci-après, l'appareil de massage selon l'invention permet de drainer la lymphe au niveau de la peau, d'améliorer par vasodilatation la micro-circulation cutanée, et d'obtenir un effet mécanique et réflexe, mais tout ceci en préservant les différents constituants de la peau, et en respectant sa visco-élasticité.

La présente invention est maintenant décrite par référence aux dessins annexés, dans lesquels :
- la figure 1 représente une vue en perspective par en-dessous d'une tête de traitement selon un premier mode d'exécution de l'invention
- la figure 2 représente une vue en élévation de la tête selon la figure 1
- la figure 3 représente une vue en élévation de la tête de traitement représentée aux figures 1 et 2
- la figure 4 représente une vue de dessous d'une tête de traitement selon un autre mode d'exécution de l'invention
- la figure 5 représente, en coupe verticale, un autre mode d'exécution de l'invention
- la figure 6 représente, en vue de dessus avec arrachement partiel, l'appareil représenté à la figure 5
- la figure 7 représente schématiquement en coupe verticale le mode d'action sur la peau de la tête de traitement d'un appareil de massage selon la présente invention
- la figure 8 représente schématiquement en coupe transversale le même mode d'action sur la peau de la tête de traitement selon l'invention.

Un appareil de massage selon l'invention comprend :
- une source de dépression, du type pompe à vide, non représentée
- et une tête de traitement 1 reliée par un tuyau non représenté, à la source de vide ou dépression envisagée précédemment ; c'est cette tête de traitement 1 qui est manipulée par l'opérateur à la surface du corps du patient, comme décrit ci-après.

La tête de traitement 1 consiste en un boîtier ayant généralement la forme d'une cloche ou d'une alvéole, et délimitant de manière interne une chambre d'aspiration 2 de la peau ; cette dernière est reliée à la source de dépression ou vide, envisagé précédemment, par un orifice 3 traversant la paroi supérieure de la chambre d'aspiration 2.

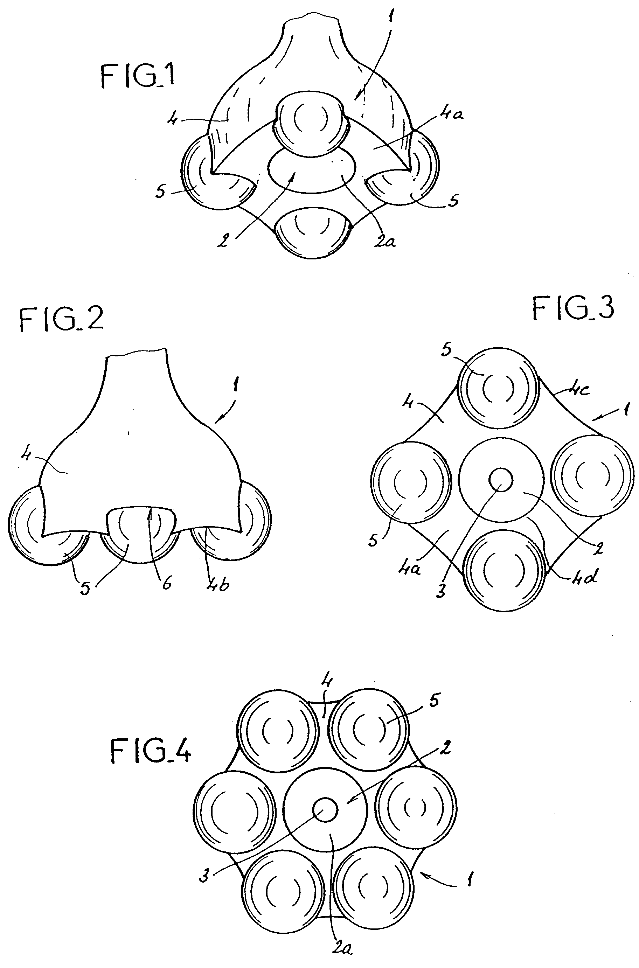

La bordure 4a de la paroi 4 du boîtier, délimitant l'ouverture 2a de la chambre d'aspiration 2, est pourvue de quatre billes 5 de massage, régulièrement distribuées sur le pourtour de l'ouverture 2a. Ces billes sont fixes en position par rapport au boîtier 1, ont un plan équatorial commun, mais sont montées libres en rotation dans toutes les directions sur la bordure 4a. Comme le montrent les figures 2 et 3, d'une part des intervalles sensiblement égaux séparent dans le plan équatorial commun les billes 5 distribuées sur le pourtour de l'ouverture 2a, et d'autre part le niveau inférieur des billes 5 déterminant la surface de roulement de la tête de traitement 1, est situé au dessous du bord libre 4b de l'ouverture 2a, de manière a ménager entre deux billes 5 consécutives un interstice 6 de pénétration partielle de la peau du patient, sous aspiration dans la chambre 9.

Comme le font apparaître les figures 1 et 3, la paroi du boîtier 1 comporte une double enveloppe 4c/4d au sein de laquelle les billes de massage 5 sont prisonnières par clipsage en position, mais pas en rotation.

Conformément à la figure 4, il est possible de prévoir et disposer un nombre supérieur de billes de massage, par exemple six billes disposées selon un hexagone.

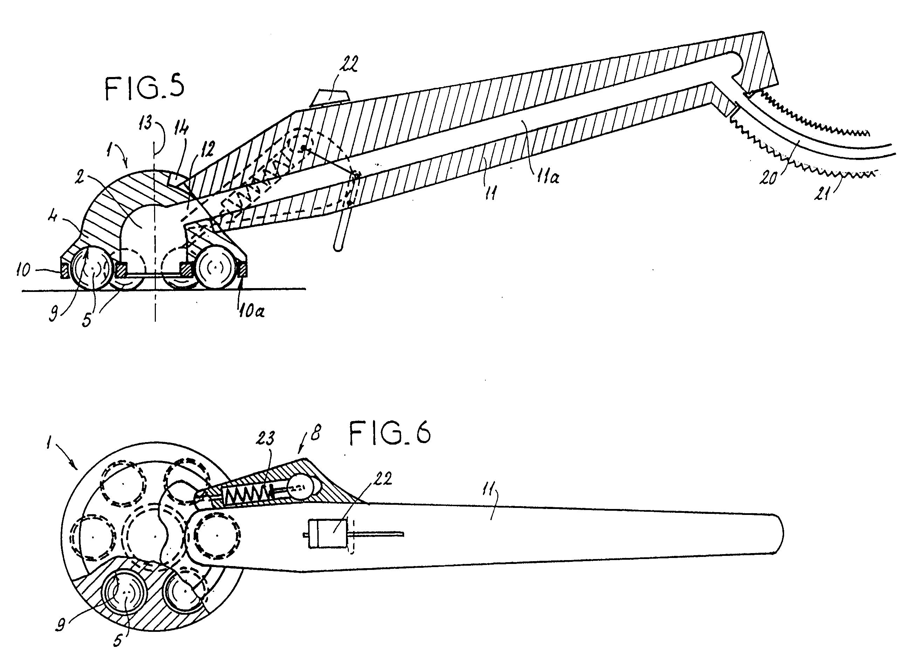

Le mode d'exécution conforme aux figures 5 et 6 diffère de celui décrit par référence aux figures 1 à 3, par les caractéristiques techniques suivantes.

Tout d'abord, la paroi 4 du boîtier délimitant la chambre 2 d'aspiration, est pleine, et comporte une pluralité de logements 9 en creux, partiellement sphériques, pour la réception des billes 5 de massage respectivement. Une platine 10, rapportée à plat par tout moyen approprié sur la face inférieure et transversale de la paroi pleine 4, comporte une pluralité d'ouvertures circulaires, en correspondance avec les logements 9 partiellement sphériques respectivement ; c'est cette platine 10 qui retient les billes 5 dans leurs logements respectifs 9. Cette platine 10 joue donc le même rôle que celui décrit pour la bordure 4a selon le mode d'exécution des figures 1 à 3.

Un manche 11 de manipulation est associé à la tête de traitement 11 ; plus précisément, cette dernière est montée à l'une des extrémités du manche 11. Ce dernier comporte un canal central 11a d'aspiration, communiquant a une extrémité avec la chambre d'aspiration 2, et à l'autre extrémité avec un flexible 20, relié à une pompe d'aspiration non représentée, le flexible étant lui-même protégé par un conduit extérieur souple 21.

Le manche 11 est articulé à la tête de traitement 1, tout en permettant une communication permanente entre le canal d'aspiration 11a et la chambre d'aspiration 2. A cette fin, la tête 1 est agencée de la manière suivante :
- une fente 12, assurant un passage de l'intérieur de la chambre 2 vers l'extérieur de la tête 1, s'étend parallèlement à la bordure 10 du boîtier, ce qui permet un déplacement en rotation du manche 11 autour de l'axe vertical 13 du boîtier ; de manière non représentée, des moyens d'étanchéité sont prévus vis-à-vis de la fente 12, de part et d'autre de la jonction de cette dernière avec le canal 11a d'aspiration du manche 11, ceci de manière à permettre une dépression suffisante dans la chambre d'aspiration 2
- la tête 1 comporte également une rainure 14, à la fois d'extension axiale par rapport au boîtier, et d'extension longitudinale dans les limites de la fente 12, toujours par rapport au boîtier ; ceci permet un déplacement en rotation du manche 11, dans un plan passant par l'axe 13 du boîtier ; de la même manière que précédemment, des moyens non représentés d'étanchéité sont prévus vis-à-vis de la rainure 14, de part et d'autre de la jonction de cette dernière avec le canal 11a d'aspiration du manche 11.

En définitive, comme le permettent les moyens décrits précédemment, le manche 11 peut être orienté par rapport à la tête de traitement 1, selon deux degrés de liberté, le premier dans un plan perpendiculaire à l'axe 13, dans un secteur angulaire limité par les deux extrémités de la fente 12, et le second dans un plan passant par l'axe 13, dans un secteur angulaire limité par les deux bordures inférieure et supérieure de la rainure ou évidement 14.

L'appareil constitué par conséquent du manche 11 et de la tête 1 comprend par ailleurs un moyen de réglage de la dépression dans la chambre d'aspiration 2, grâce au bouton de commande 22, situé sur le dessus du manche 11.

Un dispositif 8 de distribution d'un produit liquide, par exemple de traitement pharmacologique, est disposé sur l'appareil, par exemple sur le côté du manche 11 (cf figure 6), en relation avec l'intérieur de la chambre 2 d'aspiration, et en particulier la surface externe d'au moins une bille de massage 5. Un mécanisme à détente 23, pouvant être actionné par l'opérateur, permet d'exprimer le produit liquide.

Les modes d'exécution décrits précédemment sont susceptibles de nombreuses variantes, sans sortir du cadre de la présente invention. En particulier, les billes 5 de massage peuvent être constituées d'une matière ou alliage magnétique, ce qui permet d'ajouter un traitement magnétothérapique au massage effectué.

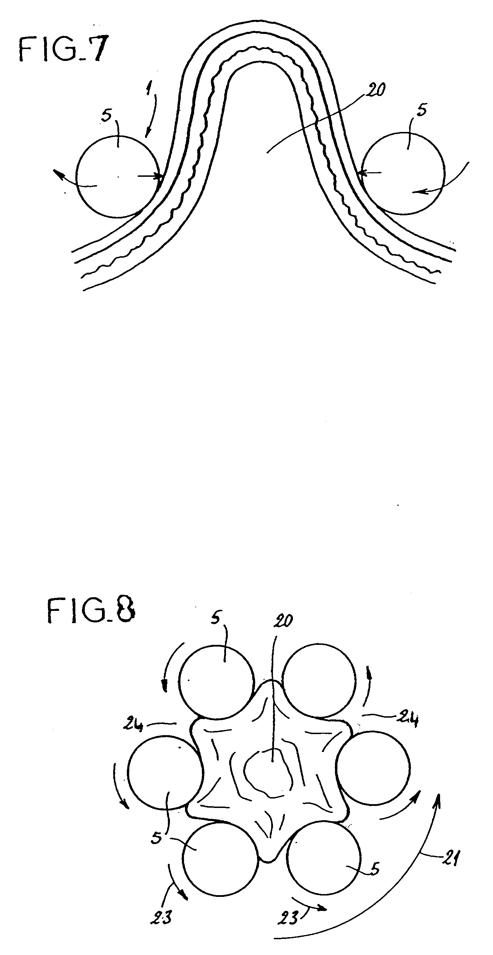

Conformément aux figures 7 et 8, dès que la chambre 2 est mise sous dépression, en appliquant la tête 1 à la surface de la peau, on obtient un pli cutané ayant une forme en cloche, pénétrant ou occupant l'ouverture 2a en direction de la chambre 2. L'opérateur déplace sur la peau la tête de traitement 1, en décrivant avec cette dernière de larges mouvements circulaires selon la flèche 21, mais sous pression importante appliquée sur la peau. Ce faisant, la face interne du pli cutané en cloche pénètre partiellement dans chaque interstice 24, entre deux billes 5 consécutives, et à l'avant de chaque bille. Par le déplacement circulaire 21 de la tête 1, les billes 5 sont animées individuellement d'un mouvement de rotation 23, dans le même sens que la rotation 21. De cette manière, le pli cutané 20 est non seulement massé à sa base, comme montré par la figure 7, mais aussi sur sa face interne et sur tout son pourtour, comme montré par la figure 8. De plus, la pénétration partielle du pli cutané, entre deux billes consécutives, permet sous l'effet du mouvement circulaire 21 d'ensemble un effet de chasse vasculaire intense. Au total, on aboutit mécaniquement, c'est-à-dire sans massage direct de la peau par l'opérateur, a une stimulation douce, vasculaire et réflexe de la peau.

Deux gammes de dépression peuvent être envisagées pour le fonctionnement d'une tête de massage selon l'invention, la première relativement basse entre 50 et 250 mbars, et la seconde relativement haute entre 250 et 400 mbars.

En dehors des applications de dermo-esthétique, et autres applications non thérapeutiques, l'appareil de massage précédemment décrit a été testé à des fins cliniques, par exemple :
- sur les troubles circulatoires des membres inférieurs, caractérisés par des sensations de jambes lourdes, parfois par un oedème sus-malléolaire vespéral
- sur des "cellulites" ou lipodystrophies.

Dans ces applications, l'étude porte sur 20 cas, tous du sexe féminin, agés de 18 à 65 ans.

Le massage a été effectué comme décrit précédemment, avec un mouvement circulaire dans le sens de la circulation lymphatique. Dans chaque cas, le nombre de séances a été de 10 en 5 semaines, et chaque séance a été divisée en deux période, à savoir une première période de 15 minutes, sous une dépression de 200 mbars, sur les zones lipodystrophiques localisées, le plus souvent dans les régions pré-trochantériennes.

Les résultats obtenus ont été évalués sur :
- l'aspect "peau d'orange", pour les lipodystrophies
- la diurèse post-massage
- l'épaisseur du pli cutané, à la face latérale externe, dans la région pré-trochantérienne
- le périmètre de la cuisse mesuré à 20 cm au-dessus de la rotule
- les sensations éprouvées par les patientes.

De ces essais, les conclusions suivantes ont pu être tirées :
1) dans 90 % des cas, on observe une disparition de la "peau d'orange"
2) dans 70 % des cas, il y a une augmentation notable de la diurèse post-massage, ce pendant 12 heures
3) dans 70 % des cas, l'épaisseur du pli cutané diminue de 15 mm
4) le périmètre de la cuisse diminue en moyenne de 15 mm
5)
   * 90 % des patientes éprouvent une impression de bien-être, de détente
   * 70 % ressentent une disparition de la sensation de lourdeur des membres inférieurs, et voient l'oedème sus-malléolaire disparaître
   * toutes trouvent le massage indolore
   * dans tous les cas, la tolérance reste parfaite
6) l'érythrose cutanée est une constante avec l'exfoliation cutanée.

## Revendications

1. Appareil de massage par décollement de la peau par dépression, comprenant une tête (1) de traitement manipulée par l'opérateur a la surface du corps, ladite tête comportant une chambre (2) d'aspiration en forme d'alvéole, reliée à une source de dépression, pour l'aspiration dans ladite chambre d'un pli cutané en forme de cloche, ladite chambre (2) d'aspiration communiquant vers la peau par une ouverture (2a) délimitée par une paroi (4) dont la bordure (4a,10) est pourvue d'une pluralité de billes (5), régulièrement distribuées sur le pourtour de ladite ouverture, montées fixes dans un même plan équatorial par rapport à la tête (1), mais montées libres en rotation, dans toutes les directions sur ladite bordure, la surface de roulement de la tête de traitement, définie par le niveau inférieur des billes, étant située à distance du bord libre (4b,10a) de l'ouverture (2a), **caractérisé en ce que** des interstices de pénétration partielle des faces internes du pli cutané en cloche sont ménagés et distribués sur le pourtour de l'ouverture (2a) de la chambre (2) d'aspiration, et sont définis, entre la surface de roulement et le bord libre de l'ouverture, par des intervalles sensiblement égaux séparant les billes (5) dans leur plan équatorial.

2. Appareil selon la revendication 1, caractérisé en ce que la paroi (4) comporte une double enveloppe (4c,4d), au sein de laquelle les billes (5) de massage sont prisonnières en position, mais pas en rotation.

3. Appareil selon la revendication 1, caractérisé en ce que la bordure (4a, 10) de la paroi comporte au moins quatre billes (5) de massage.

4. Appareil selon la revendication 1, caractérisé en ce que la paroi (4) est pleine, et comporte une pluralité de logements (9) en creux, partiellement sphériques, pour la réception des billes (5) de massage respectivement, et en ce qu'une platine (10) rapportée à plat sur la face transversale de la paroi pleine, comportant une pluralité d'ouvertures circulaires correspondant aux billes (5) respective ment, retient ces dernières dans leurs logements respectifs.

5. Appareil selon la revendication 1, caractérisé en ce qu'il comprend un manchon (11) de manipulation, à une extrémité duquel est montée la tête (1) de traitement.

6. Appareil selon la revendication 5, caractérisé en ce que le manche (11) Comporte un canal central (11a) d'aspiration communiquant avec la chambre (2) d'aspiration de la tête (1) de traitement.

7. Appareil selon la revendication 5, caractérise en ce que le manche (11) de manipulation est articulé sur la tête (1) de traitement.

8. Appareil selon les revendications 6 et 7, caractérisé en ce que la tête (1) comporte une fente (12) d'extension parallèle à la bordure (10) du boîtier, permettant un déplacement en rotation du manche (11) autour de l'axe (13) dudit boîtier, des moyens d'étanchéité étant prévus vis-à-vis de la fente (12), de part et d'autre de la jonction de cette dernière avec le canal (11a) d'aspiration du manche (11).

9. Appareil selon les revendications 6 et 7, caractérisé en ce que la tête (1) comporte une rainure (14) d'extension axiale par rapport au boîtier, permettant un déplacement en rotation du manche (11), dans un plan passant par l'axe (13) du boîtier, des moyens d'étanchéité étant prévus vis-à-vis de la rainure (14), de part et d'autre de la jonction de cette dernière avec le canal (11a) d'aspiration du manche (11).

10. Appareil selon la revendication 1, caractérisé en ce qu'il comprend un dispositif (8) de distribution d'un produit liquide, en relation avec l'intérieur de la chambre (2) d'aspiration, et la surface externe d'au moins une bille de massage (5).

11. Appareil selon la revendication 1, caractérisé en ce que la dépression générée dans la chambre d'aspiration par la source de dépression est au plus égale à 300 mbars.

## Claims

1. Apparatus for massaging by pulling away the skin using reduced pressure, comprising a treatment head (1) manipulated by the operator on the surface of the body, said head including a suction chamber (2) in the shape of an air sac, connected to a reduced-pressure source, for suction of a cutaneous fold in the shape of a bell into said chamber, said suction chamber (2) communicating with the skin via an opening (2a) delimited by a wall (4) whose border (4a,10) is provided with a plurality of balls (5), regularly distributed on the perimeter of said opening, mounted fixed in the same equatorial plane with respect to the head (1), but mounted so as to rotate freely in all directions on said border, the rolling surface of the treatment head, defined by the lower level of the balls, being situated at a distance from the free edge (4b,10a) of the opening (2a), characterized in that gaps for partial penetration of the internal faces of the bell-shaped cutaneous fold are formed and distributed on the perimeter of the opening (2a) of the suction chamber (2), and are defined, between the rolling surface and the free edge of the opening, by approximately equal intervals separating the balls (5) in their equatorial plane.

2. Apparatus according to Claim 1, characterised in that the wall (4) includes a double envelope (4c,4d), within which the massage balls (5) are confined in position, but not in rotation.

3. Apparatus according to Claim 1, characterised in that the border (4a,10) of the wall includes at least four massage balls (5).

4. Apparatus according to Claim 1, characterised in that the wall (4) is solid, and includes a plurality of hollow, partially spherical housings (9) for receiving the massage balls (5) respectively, and in that a plate (10) attached flat onto the transverse face of the solid wall, including a plurality of circular openings corresponding to the balls (5) respectively, retains the latter in their respective housings.

5. Apparatus according to Claim 1, characterised in that it comprises a manipulation handle (11), at one end of which the treatment head (1) is mounted.

6. Apparatus according to Claim 5, characterised in that the handle (11) includes a central suction channel (11a) communicating with the suction chamber (2) of the treatment head (1).

7. Apparatus according to Claim 5, characterised in that the manipulation handle (11) is articulated onto the treatment head (1).

8. Apparatus according to Claims 6 and 7, characterised in that the head (1) includes a slit (12) of extension parallel to the border (10) of the case, allowing the handle (11) to move in rotation around the axis (13) of said case, sealing means being provided opposite the slit (12), on either side of the junction of the latter with the suction channel (11a) of the handle (11).

9. Apparatus according to Claims 6 and 7, characterised in that the head (1) includes a groove (14) of axial extension with respect to the case, allowing the handle (11) to move in rotation, in a plane passing through the axis (13) of the case, sealing means being provided opposite the groove (14), on either side of the junction of the latter with the suction channel (11a) of the handle (11).

10. Apparatus according to Claim 1, characterised in that it comprises a device (8) for distributing a liquid product, in contact with the inside of the suction chamber (2), and the external surface of at least one massage ball (5).

11. Apparatus according to Claim 1, characterised in that the reduced pressure generated in the suction chamber by the reduced-pressure source is at most equal to 300 mbar.

## Patentansprüche

1. Gerät zur Massage durch Ablösung der Haut mittels Unterdruck, umfassend einen Kopf (1) zur Behandlung, der durch den Benutzer an der Oberfläche des Körpers manipuliert wird, wobei der Kopf eine Ansaugkammer (2) in Form einer Alveole umfaßt, die mit einer Unterdruckquelle verbunden ist, um unter der besagten Kammer eine Hautfalte in Form der Glocke anzusaugen, wobei die Kammer (2) zum Ansaugen mit der Haut über eine Öffnung (2a) in Verbindung steht, die über eine den Rand (4a, 10) bildende Wand (4) begrenzt ist, die mit einer Mehrzahl von Kugeln (5) versehen ist, die gleichmäßig über die Fläche der Öffnung verteilt sind und in einer gemeinsamen Äquatorialebene in Bezug auf den Kopf (1) fest montiert sind, aber in allen Richtungen über dem Rand frei drehbar sind, wobei die Oberfläche des Kopfes zur Behandlung, die durch das untere Niveau der Kugeln gebildet ist, in einem Abstand von dem freien Rand (4b, 10a) der Öffnung (2a) angeordnet ist, dadurch gekennzeichnet, daß Zwischenräume der teilweisen Penetration der internen Flächen der Hautfalten in der Glocke über die ganze Öffnung (2a) in der Saugkammer (2) angeordnet und verteilt sind und zwischen der Kugelfläche und dem freien Rand der Öffnung durch deutliche Zwischenräume festgelegt sind, die zugleich die Kugeln (5) in ihrer Äquatorialebene trennen.

2. Vorrichtung gemäß Anspruch 1, dadurch gekennzeichnet, daß die Wand (4) einen doppelten Mantel (4c, 4d) umfaßt, innerhalb dessen die Kugeln (5) zur Massage in Position, aber drehbar gehalten sind.

3. Vorrichtung gemäß Anspruch 1, dadurch gekennzeichnet, daß der Rand (4a, 10) der Wand wenigstens vier Kugeln (5) zur Massage umfaßt.

4. Vorrichtung gemäß Anspruch 1, dadurch gekennzeichnet, daß die Wand (4) eben ist und eine Mehrzahl von teilweise sphärischen Hohllagern (9) umfaßt, um die betreffenden Kugeln (5) zur Massage aufzunehmen, und daß eine ebene Platine (10) auf die Querfläche der ebenen Wand aufgesteckt ist, die eine Mehrzahl von kreisförmigen Öffnungen umfaßt, die mit den betreffenden Kugeln (5) korrespondieren und die letztere in ihren betreffenden Lagern zurückhalten.

5. Vorrichtung gemäß Anspruch 1, dadurch gekennzeichnet, daß sie einen Bedienungsstutzen (11) umfaßt, an dessen einem Ende der Kopf (1) zur Behandlung montiert ist.

6. Vorrichtung gemäß Anspruch 5, dadurch gekennzeichnet, daß der stutzen (11) einen zentralen Saugkanal (11a) umfaßt, der mit der Saugkammer (2) des Kopfes (1) zur Behandlung in Verbindung steht.

7. Vorrichtung gemäß Anspruch 5, dadurch gekennzeichnet, daß der Bedienungsstutzen (11) gelenkig mit dem Kopf (1) zur Behandlung verbunden ist.

8. Vorrichtung gemäß Anspruch 6 oder 7, dadurch gekennzeichnet, daß der Kopf (1) einen Schlitz (12) mit einer Ausdehnung umfaßt, die parallel zum Rand (10) des Gehäuses ist und eine Verdrehung des Stutzens (11) um die Achse (13) des Gehäuses ermöglicht, das Mittel zur Abdichtung gegenüber dem Schlitz (12) beidseits der Kreuzung des letzteren mit dem Saugkanal (11a) des Stutzens (11) vorgesehen sind.

9. Vorrichtung gemäß Anspruch 6 und 7, dadurch gekennzeichnet, daS der Kopf (1) eine Rille (14) umfaßt, die sich axial in Bezug auf das Gehäuse erstreckt und eine Verdrehung des Stutzens (11) in einer Ebene erlaubt, die durch die Achse (13) des Gehäuses verläuft, daS die Mittel zur Abdichtung gegenüber der Rille (14) beidseits der Kreuzung der letzteren mit dem Saugkanal (11a) des Stutzens (11) vorgesehen sind.

10. Vorrichtung gemäß Anspruch 1, dadurch gekennzeichnet, daß sie eine Einrichtung (8) zum Verteilen eines flüssigen Produktes in Bezug auf das Innere der Saugkammer (2) und auf die äußere Oberfläche wenigstens einer Massagekugel (5) umfaßt.

11. Vorrichtung gemäß Anspruch 1, dadurch gekennzeichnet, daß der Druck innerhalb einer Saugkammer durch eine Saugquelle mit mehr als 300 mbar erzeugt wird.
